# EUROPEAN PATENT APPLICATION

(11) **EP 1 844 759 A1**
(43) Date of publication of application: **17.10.2007**
(21) Application number: 06380064.3
(22) Date of filing: 31.03.2006
(51) Int. Cl.: A61K 8/73, A61K 8/86, A61K 8/898, B01F 17/00, A61Q 5/12, A61Q 19/10, A61Q 13/00

(54) **Compositions containing fragrance or perfume**

(71) Applicant: KAO CORPORATION, S.A., 08210 Barbera del Vallés - Barcelona (ES)
(72) Inventor: Abe, Hiroshi, 08210 Barbera del Vallés - Barcelona (ES); Bueno Perisé, Agusti, 08210 Barbera del Vallés - Barcelona (ES); Castán Barberán, Pilar, 08210 Barbera del Vallés - Barcelona (ES); Minguet Bonvehi, Maria, 08210 Barbera del Vallés - Barcelona (ES); Nogués López, Blanca, 08210 Barbera del Vallés - Barcelona (ES)
(74) Representative: Renken, Joachim

(57) **Abstract**

The invention relates to a composition containing fragrance or perfume containing particular polysaccharide derivatives and branched polyglycerol-modified silicone. In particular, it provides an aqueous composition comprising:
(a) a fragrance material or a perfume,
(b) a particular polysaccharide derivative,
(c) a branched polyglycerol-modified silicone
(d) one or more surfactants.

This composition containing fragrance or perfumecan be incorporated into detergent or cleaning formulations such as laundry detergents, fabric softeners, soaps, personal care products, such as shampoos, body washes and the like, as well as numerous other products, to enhance fragrance or perfume performance.

The composition containing fragrance or perfume provides controlled release from the substrate where it has been deposited over an extended period of time.

## Description

### Technical field

This invention relates to compositions containing fragrance or perfume in addition to particular polysaccharide derivatives and branched polyglycerol-modified silicone. Said compositions could be incorporated in personal washing and hair-conditioning compositions, laundry detergent compositions and rinse conditioner compositions for fabric softening.

### Prior Art

Fragrances (any substance, natural or synthetic, used to impart an odour to a product) or perfumes (mixture of fragrant essential oils and aroma compounds, fixatives, and solvents used to give the human body, objects, and living spaces a lasting and pleasant smell) are frequently incorporated in detergents and other cleaning products to give a pleasant odour during use of the cleaning product and to mask the inherent smell of the soap or other surfactant present in the cleaning product.

Upon storage in a cleaning composition, fragrances and perfumes can be altered through interactions and/or reactions with the other components of the composition.

Due to their volatile nature, the fragrant compounds tend to be dissipated with time, particularly the most volatile compounds which are often associated with perceived freshness.

Moreover, when used, such as during washing of fabrics with a laundry detergent, most of the perfume is also lost in the aqueous phase during the washing cycle and/or the rinsing cycle. It has been recognised as desirable that the fragrance should survive storage in the cleaning composition and also survive the cleaning process and should be deposited on the fabric, so that fabrics laundered with a detergent containing the fragrance or softened with a fabric softener containing the fragrance should have the pleasant odour of the fragrance.

Furthermore, once adsorbed onto the targeted surface, for example fabrics or hair or skin, the fragrance tends to be dissipated very quickly. A long-lasting odour, slowly emitted from the substrate (surface, fabric, skin, hair, etc.) is a desired property of the fragrances or perfumes incorporated in the detergent or cleaning compositions, which is often described as substantivity, tenacity or longevity.

To stable incorporate fragrances or perfumes in aqueous solutions containing other ingredients, different techniques have been applied. Micro-encapsulation has been widely described in the prior art and used to a large extent, first of all as a means to improve the stability of volatile and labile ingredients such as fragrances or perfumes, by protecting them from all kinds of possible aggressions or degradation processes such as oxidation, but also as also a means to provide release of an active ingredient, which is spread out over a more or less extended period of time, instead of being instantaneous. Micro-encapsulation is defined in the "Kirk-Othmer Encyclopedia of Chemical Technology", third edition, volume 15, published by Wiley-Interscience (ISBN 0-471-02095-3), as a process in which tiny particles or droplets are surrounded by a coating to give small capsules with many useful properties.

The absorption of perfume onto polymeric carrier materials, capable of improving the substantivity of perfume on substrates, is also the subject of some disclosures from the prior art, in particular in the patent literature. More particularly the use of combined polymeric carriers has been described within the framework of perfume encapsulation, with the aim of improving the deposition of perfume on a substrate, in an application. As an example, US-B-6194375 discloses organic polymer particles comprising perfume absorbed therein, and wherein a further polymer incorporating free hydroxyl groups is attached at the exterior of the particles. In a detergent application, the latter polymer serves to promote deposition of the particles from a wash or rinse liquor during a washing cycle. Similarly, US-B-6329057 describes polymeric particles comprising a hydrophobic organic matrix and, located at the exterior, free cationic groups and a further polymer which comprises free hydroxy groups.

To stable incorporate oil components and/or fragrances or perfumes in aqueous solutions containing other ingredients also water-in-oil or oil-in-water emulsions have been described. According to the "Kirk-Othmer Encyclopedia of Chemical Technology", third edition, volume 8, published by Wiley-interscience (ISBN 0-471-02095-3), an emulsion is a mixture of two or more immiscible liquids one being present in the other in the form of droplets.

As an example, EP-A-1369102 describes a process for the preparation of an o/w emulsion, which comprises mixing together
a) a high molecular compound carrying at least on side chains thereof groups represented by the following formula (1):

   - (OX) n-E²-R (1)

   wherein
   - "X"s may be the same or different and each independently represents a linear or branched, divalent, saturated C₁-C₆ hydrocarbon group,
   - n stands for a number of from 5 to 300,
   - E² represents an ether bond or an oxycarbonyl group (-OCO- or -COO-), and
   - R represents a linear or branched, C₄-C₃₀ alkyl group which may be substituted by hydroxyl group(s),
b) an aqueous solution of a water-soluble polyol, and
c) a hydrophobic compound,
and diluting the resulting mixture with water.

In EP-A-1369102 it is indicated that examples of hydrophobics compounds are higher alcohols, sterols, silicones, fluorine-containing oils and oil components including fragrances.

Furthermore, in EP-A-1369102 it is stated that the emulsions obtained in accordance with the process described exhibit good stability in combination with surfactants systems and, additionally, are excellent in touch feeling to skin and good in stability.

However, nothing from the disclosure of EP-A-1369102 suggests that said o/w emulsion could be useful for improving the substantivity of a perfume on substrates.

Preferred high molecular compounds according to EP-A-1369102 are the polysaccharides described in EP-A-1191039 (WO-A-0073351). Said polysaccharides can be used as thickening and stabilizing agent for toiletry products and cleaning agents. Furthermore, EP-A-1191039 teaches personal care formulations containing the specific polysaccharide derivatives by themselves, but not in the form of oil-in-water emulsions.

Again, nothing from the disclosure of EP-A-1191039 suggests that said polysaccharide derivatives could be useful for improving the substantivity or longevity of a perfume on substrates.

WO-A-2004024114 describes compositions comprising
i) fragrance compositions, and
ii) silicone-in-water emulsions comprising at least one surfactant,
wherein the dispersed silicone has a viscosity of 100 million mm²s⁻¹ or more and a particle size of less than 2 mm.

According to the teaching of WO-A-2004024114 such compositions provide improved release of fragrance when applied to the hair and improved deposition of the fragrance on the hair. Although the polysiloxanes according to WO-A-2004024114 can have a small amount of branching (e.g. less than 2 mole % of the siloxane units), they are preferably completely linear.

Finally, EP-A-1489128 teaches branched polyglycerol-modified silicone, a method for producing the same and a cosmetic containing the same. It is stated that said branched polyglycerol-modified silicones remain adsorbed on fabrics, hair or skin higher than conventional modified silicone compounds (amino-modified silicone), even when the fabrics are treated in a very dilute concentration.

But nothing from the disclosure of EP-A-1489128 suggests that said branched polyglycerol-modified silicones could be useful either for a stable incorporation of fragrances or perfumes in aqueous solutions or for improving the substantivity or longevity of the perfume on substrates.

It can be concluded from the existing prior art that the industry still requires improvements in the field of the controlled release of stable incorporated fragrances or perfumes and an efficient deposition of them on a substrate so as to obtain a sustained release from such a surface, is still needed.

### Summary of the invention

The present invention offers an efficient solution to the problems encountered in the prior art, by providing a composition containing a fragrance or perfume, a particular polysaccharide derivative and a branched polyglycerol-modified silicone and one or more surfactants. Said composition of the invention protect the perfume during its use and storage, protecting it from interactions with other constituents when used to perfume a functional consumer product and extend the gradually release of said perfume, enhancing the odour longevity (sustantivity), slowly emitted from the substrate.

It has now been found, unexpectedly, that personal care and household compositions containing the composition according to the invention exhibit a significantly improvement of the fragrance performance in said compositions. Furthermore, the lasting properties of the fragrance are also significantly improved.

Although the present invention should not be regarded as bound to the theory discussed hereinafter, it is believed that those beneficial effects may be attributable to the synergistic effect between the polysaccharide derivative and a branched polyglycerol-modified silicone, which enhance the deposition of the fragrance of perfume on a substrate, preferably on natural and synthetic fibres and skin, extending gradually the release of said perfume, enhancing the odour longevity (substantivity), slowly emitted from the substrate.

According to the present invention, synthetic fibres are fibres of chemical origin, which are defined as fibres obtained by physical or chemical processes in production means, which comprises cellulosic and synthetic fibres as described in the "Kirk-Othmer Encyclopedia of Chemical Technology", third edition, volume 10, published by Wiley-Interscience (ISBN 0-471-02095-3). Examples of fibres of chemical origin are:
- acrylic, polyamide, polyester and polyurethane; while fibres of synthetic origin are,
- acetate and rayon, which are of cellulosic origin.

Examples of fibres of natural origin are:
- cotton, from vegetable origin;
- camel, cashmere, silk and wool, which are of animal origin; and
- human hair.

Thus, the present invention provides a (aqueous) composition comprising
(a) a fragrance material or a perfume;
(b) a polysaccharide derivative derived from a polysaccharide, or a derivative thereof, by substituting a part or all of the hydrogen atoms of the hydroxyl groups of the polysaccharide or the derivative thereof with substituent group (A):
   (A) groups represented by the following formula:

   -E¹-(OX)ₙ-E²-R (1)

   wherein
   - E¹ represents a linear or branched, divalent saturated C₁-C₆ hydrocarbon group which may be substituted with one or more hydroxyl groups or oxo groups,
   - X may be the same or different and each independently represent a divalent saturated C₁-C₆ hydrocarbon group, and n represents a number of from 8 to 300, preferably 8 to 50, more preferably 10 to 20,
   - E² represents an ether bond or an oxycarbonyl group (-OCO- or -COO-), and
   - R represents a linear or branched C₄-C₃₀ alkyl group which may be substituted with one or more hydroxyl groups,
   - the H(s) of one or more hydroxy groups in each of said substituted groups (A) may be further substituted with said substituted groups (A);
(c) a branched polyglycerol-modified silicone; and
(d) one or more surfactants.

The subject matter of the present invention also includes the personal care compositions and the household compositions, which comprise the mentioned composition.

The present invention also provides the use of the mentioned composition for enhancing the fragrance or perfume longevity of personal care compositions and household compositions.

### Detailed description of the invention

### Fragrance or Perfume

By fragrance or perfume is meant any organic substance or composition which has a desired olfactory property and is essentially non-toxic. Such substances or compositions include all fragrances or perfumes that are commonly used in perfumery or in household compositions (laundry detergents, fabric softeners, soaps, all-purpose cleaners, bathroom cleaners, floor cleaners) or personal care compositions. The compounds involved may be natural, semi-synthetic or synthetic in origin.

Preferred fragrances or perfumes may be assigned to the classes of substance comprising the hydrocarbons, aldehydes or esters. The fragrances or perfumes also include natural extracts and/or essences, which may comprise complex mixtures of constituents, i.e. fruits such as almond, apple, cherry, grape, pear, pineapple, orange, lemon, strawberry, raspberry and the like; musk, flower scents such as lavender, jasmine, lily, magnolia, rose, iris, carnation and the like; herbal scents such as rosemary, thyme, sage and the like; woodland scents such as pine, spruce, cedar and the like.

Non limitative examples of synthetic and semi-synthetic fragrances and perfumes are: 7-acetyl-1,2,3,4,5,6,7,8-octahydro-1,1,6,7-tetramethylnaphthalene, α-ionone, β-ionone, γ-ionone, α-isomethylionone, methylcedrylone, methyl dihydrojasmonate, methyl 1,6,10-trimethyl-2,5,9-cyclododecatrien-1-yl ketone, 7-acetyl-1,1,3,4,4,6-hexamethyltetralin, 4-acetyl-6-tert-butyl-1,1-dimethylindane, hydroxyphenylbutanone, benzophenone, methyl b-naphthyl ketone, 6-acetyl-1,1,2,3,3,5-hexamethylindane, 5-acetyl-3-isopropyl-1,1,2- ,6-tetramethylindane, 1-dodecanal, 4-(4-hydroxy-4-methylpentyl)-3-cyclohex-ene-1-carboxaldehyde, 7-hydroxy-3,7-dimethyloctanal, 10-undecen-1-al, isohexenylcyclohexylcarboxaldehyde, formyltricyclodecane, condensation products of hydroxycitronellal and methyl anthranilate, condensation products of hydroxycitronellal and indole, condensation products of phenylacetaldehyde and indole, 2-methyl-3-(para-tert-butylphenyl)propionaldehyde, ethylvanillin, heliotropin, hexylcinnamaldehyde, amylcinnamaldehyde, 2-methyl-2-(isopropylphenyl)propionaldehyde, coumarin, γ-decalactone, cyclopentadecanolide, 16-hydroxy-9-hexadecenoic acid lactone, 1,3,4,6,7,8-hexahydro-4,6,6,7,8,8-hexamethylcyclopenta-g-benzopyran, β-naphthol methyl ether, ambroxane, dodecahydro-3a,6,6,9a-tetramethylnaphtho[2,1b]furan, cedrol, 5-(2,2,3-trimethylcyclopent-3-enyl)-3-methylpentan-2-ol, 2-ethyl-4-(2,2,3-trimethyl-3-cyclopenten-1-yl)-2-buten-1-ol, caryophyllene alcohol, tricyclodecenyl propionate, tricyclodecenyl acetate, benzyl salicylate, cedryl acetate, and tert-butylcyclohexyl acetate.
Particular preference is given to the following:
hexylcinnamaldehyde, 2-methyl-3-(tert-butylphenyl)propionaldehyde, 7-acetyl-1,2,3,4,5,6,7,8-octahydro-1,1,6,7-tetramethylnaphthalene, benzyl salicylate, 7-acetyl-1,1,3,4,4,6-hexamethyltetralin, para-tert-butylcyclohexyl acetate, methyl dihydrojasmonate, (β-naphthol methyl ether, methyl g-naphthyl ketone, 2-methyl-2-(para-isopropylphenyl)propionaldehyde, 1,3,4,6,7,8-hexahydro-4,6,6,7,8,8-hexamethylcyclopenta-g-2-benzopyran, dodecahydro-3a,6,6,9a-tetramethylnaphtho[2,1b]furan, anisaldehyde, coumarin, cedrol, vanillin, cyclopentadecanolide, tricyclodecenyl acetate and tricyclodecenyl propionates.

Other fragrances are essential oils, resinoids and resins from a large number of sources, such as, for example, Peru balsam, olibanum resinoid, styrax, labdanum resin, nutmeg, cassia oil, benzoin resin, coriander, and lavandin.

Further suitable fragrances include: phenylethyl alcohol, terpineol, linalool, linalyl acetate, geraniol, nerol, 2-(1,1-dimethylethyl)cyclo-hexanol acetate, benzyl acetate, and eugenol.

The fragrances or perfumes can be used as single substances or in a mixture with one another.

Perfumes frequently include solvents or diluents, for example: ethanol, isopropanol, diethylene glycol monoethyl ether, dipropylene glycol, diethyl phthalate and triethyl citrate.

### The polysaccharide derivative

Component (b) of the composition of the present invention is a polysaccharide derivative which is derived from a polysaccharide or a polysaccharide derivative by substituting a part or all of the hydrogen atoms of said polysaccharide (derivative) with the substituent group (A) as defined above. This polysaccharide derivative is disclosed in EP 1 369 102 A2, particularly pages 3, line 1 to page 6, line 25, the disclosure of which is incorporated by reference.

Examples of the polysaccharide or its derivative usable in the present invention include polysaccharides such as cellulose, guar gum, starch, pullulan, dextran, fructan, mannan (glucomannan), agar, carrageenan, chitin, chitosan, pectin, alginic acid, and hyaluronic acid; and their derivatives having substituent groups such as methyl groups, ethyl groups, hydroxyethyl groups, hydroxypropyl groups or the like. These substituent groups can be introduced either alone or in combination into constituent monosaccharide residual groups. Illustrations of the polysaccharide derivative are hydroxyethylcellulose, hydroxyethyl ethylcellulose, hydroxyethylguar gum, hydroxyethyl starch, methylcellulose, methylguar gum, methylstarch, ethylcellulose, ethylguar gum, ethylstarch, hydroxypropylcellulose, hydroxypropylguar gum, hydroxypropyl starch, hydroxyethylmethylcellulose, hydroxyethylmethylguar gum, hydroxyethyl methyl starch, hydroxypropyl methylcellulose, hydroxypropyl methylguar gum, and hydroxypropyl methylstarch. Among these polysaccharides and derivatives thereof, cellulose, starch, hydroxyethylcellulose, methylcellulose, ethylcellulose and hydroxypropylcellulose are preferred, with hydroxyethylcellulose being particularly preferred. The weight average molecular weight of these polysaccharides or their derivatives is preferably in a range of from 10,000 to 10,000,000, more preferably in a range of from 100,000 to 5,000,000, even more preferably in a range of from 200,000 to 2,000,000.

The degree of substitution by the substituent groups (A) in the substituted polysaccharide according to the present invention is preferably in a range of from 0.0001 to 1.0, more preferably in a range of from 0.0005 to 0.5, even more preferably in a range of from 0.001 to 0.1 per constituent monosaccharide residual group.

As noted above, not only the hydroxyl groups of the constituent monosaccharide residual group, but also the hydroxyl groups of said substituents (A), if present at all, may be further substituted with the groups (A). The hydroxyl groups of said substituents (A) may be further substituted with up to 5, preferably up to 3, more preferably 1 or 0 substituents (A), which are preferably identical in their chemical nature with respect to the substituent (A) that is directly linked to the constituent monosaccharide residual group, with the exception that their respective hydroxyl groups are not further substituted. However, it is most preferred that only the hydroxyl groups of the constituent monosaccharide residual group are substituted.

In addition to the substituent groups (A), the polysaccharide derivative may be further substituted with one ore more groups selected from the following groups (B), (C) and (D):
(B) a C₁₋₅ sulfoalkyl group or salt thereof, each of which may be substituted with one or more hydroxyl groups,
(C) a C₂₋₆ carboxyalkyl group or salt thereof, each of which may be substituted with one or more hydroxyl groups, and
(D) a group represented by the following formula (II):

   D¹-N(+)R¹R²R³ X(-) (II)

   wherein
   - D¹ represents a linear or branched, divalent, saturated C_{I}-₆ hydrocarbon group which may be substituted with one or more hydroxyl groups
   - R¹, R² and R³ may be the same or different and each independently represent a linear or branched C₁₋₃ alkyl group which may be substituted with one or more hydroxyl groups and Xrepresents hydroxyl ion, a halogen ion or an organic acid ion,
in which each of the hydrogen atom(s) of one or more hydroxyl groups in these groups may be further substituted with the group (A), (B), (C) or (D).

Examples of R¹, R² and R³ in each cationic substituent group (D) can include methyl, ethyl, propyl and 2-hydroxyethyl, with methyl and ethyl being preferred.

Examples of the halogen ion represented by X⁻ in each cationic substituent group (D) can include a chloride ion, a bromide ion and an iodide ion. Examples of the organic acid ion can include CH₃COO⁻, CH₃CH₂COO⁻ and CH₃(CH₂)₂COO⁻. Preferred examples of X- include a hydroxide ion, a chloride ion and a bromide ion.

The degree of substitution by these cationic substituent groups (D) is preferably in a range of from 0 to 0.5, even more preferably in a range of from 0 to 0.3 per constituent monosaccharide residual group.

It is further preferred that the recurring unit of the polysaccharide derivative according to the present invention is represented by the formula (3): 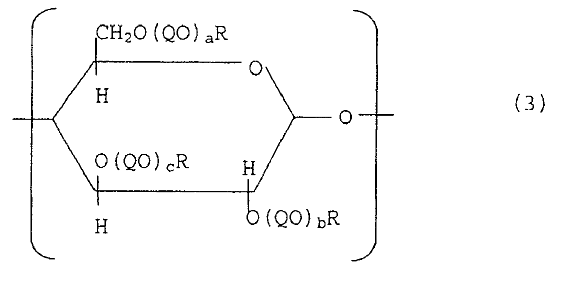 wherein
- R may be the same or different and each independently represents a group selected from
   i) hydrogen, methyl, ethyl, hydroxyethyl or hydroxypropyl
   ii) a substituent group (A) of formula (1)

      -E¹-(OX)ₙ-E²-R (1)

      wherein E¹, X, E² and R have the same meaning as defined before;
   and wherein,
- Q may be the same or different and each independently represents a C₂-C₄ alkylene group
- a, b, and c may be the same o-r different and each independently represent a number from 0 to 10
- the groups QO and R, as well as a, b, and c in the same recurring unit or in the different recurring units may be the same or different,
- the H(s) of one or more hydroxy groups in each of said substituted groups (A) may be further substituted with the groups said substituted groups (A) .

The substitution of the polysaccharide or the derivative thereof with one or more of the substituent groups (A) to (D), namely, polyoxyalkylenization, sulfoalkylation, carboxyalkylation or cationization, can be carried out by the process disclosed in EP-A-1191039.

In a particularly preferred embodiment, the polysaccharide derivative is obtained by reacting hydroxyethylcellulose with R-(OC2H4)n-O-CH2CHOCH2, wherein R is an alkyl residue with 8 to 18 carbon atoms, preferably 10 to 14 carbon atoms, most preferably 12 carbon atoms, and wherein n is a number in the range of 8 to 18, more preferably 10 to 14, most preferably 12. The preferred polysaccharide derivative has a preferred degree of substitution with hydroxyethyl groups in the range of 1 to 3 per monosaccharide unit, and a preferred degree of substitution with the polyoxyalkylene-containing substituents in the range of 0.005 to 0.05 per monosaccharide unit. Most preferred is the product of Preparation Example 11 of EP 1 369 102 A1.

### The branched polyglycerol-modified silicone

Component (c) of the composition of the present invention is a branched polyglycerol-modified silicone. This silicone is disclosed in EP 1 489 128 A1, particularly pages 4, line 14 to page 13, line 52, the disclosure of which is incorporated by reference.

Silicones with organic groups introduced into the ends and side chains are known as modified silicones.

They are categorized into four main types, according to the bonding position of the organic groups:
- side-chain type: organic groups are introduced into some of the side chains of the polysiloxane
- single-end type: organic groups are introduced into one end of the polysiloxane
- dual-end type: organic groups are introduced in both ends of the polysiloxane
- side-chain dual-ended type: organic groups are introduced into some of the side chains and both ends of the polysiloxane

The branched polyglycerol-modified silicone according to the invention has at least one branched polyglycerol chain containing at least one branched glycerol group represented by the following formula (4) and being connected via a connecting group to a silicon atom of the silicone; 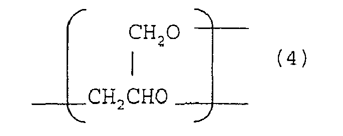 wherein two oxygen atoms, independently of each other, bind to a glycerol or glycidol group represented by formula (4) above or the following formula (5), (6), or (7).

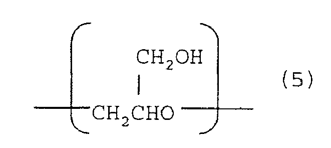

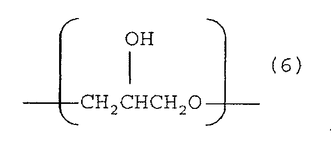

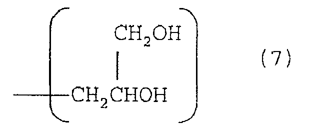

According to the invention, the branched polyglycerol chain has one or more branched glycerol groups represented by formula (4) [hereinafter, referred to as group (4)] as a branching unit. The structure of the branched polyglycerol chain includes:
- "a" groups (4),
- "b" glycidol groups represented by formula (5) [hereinafter, referred to as group (5)],
- "c" glycerol groups represented by formula (6) [hereinafter, referred to as group (6)), and
- "d" glycerol groups represented by formula (7) [hereinafter, referred to as group (7)]
as a terminal unit, which are bound to each other.

In the branched polyglycerol chain, groups (4), (5), and (6) may be bound to each other in any order. The oxygen atom in formula (5) binds to the glycerol group or glycidol group represented by formula (4), (5), (6) or (7); and the oxygen atom in formula (6) binds to a glycerol or glycidol group represented by formula (4), (5), (6) or (7). The branched polyglycerol chain has a more developed branched structure as the number of groups (4) increases and has a group (7) at the end of each branched chain.

The fact that the branched polyglycerol-modified silicone according to the invention contains one or more branching units of group (4) can be demonstrated easily by the presence of the peaks inherent in group (4) in a ¹³C-NMR spectrum. Preferably, the mean number of group (4) units per a branched polyglycerol chain is 1 or more.

According to the invention, the mean total number (a+b+c+d) of groups (4), (5), (6), and (7) per a branched polyglycerol chain is determined by the NMR analysis or comparison of the molecular weight with that of precursor silicone, and is preferably 3 or more, more preferably 3 to 201, even more preferably 3 to 101, even more preferably 3 to 51, and even more preferably 3 to 21.

The degree of branching in the branched polyglycerol chain, a/(a+b+c+d), is preferably 0.05 to 0.5, even more preferably 0.10 to 0.5, and even more preferably 0.25 to 0.5.

Groups (4), (5), (6), and (7) may be bound to each other in any order in the branched polyglycerol chain.

The number of groups (4) (i.e., a) in each branched polyglycerol chain is preferably 1 to 100, more preferably 2 to 100, even more preferably 2 to 50, even more preferably 2 to 25, and even more preferably 2 to 10. The number of groups (7) (i.e., d) in each branched polyglycerol chain is preferably 2 to 101, more preferably 3 to 101, even more preferably 3 to 51, even more preferably 3 to 26, and even more preferably 3 to 11. The number of groups (5) (i.e., b) and groups (6) (i.e., c) maybe the same or different from each other and are preferably 0 to 198, more preferably 0 to 196, even more preferably, 0 to 96, even more preferably, 0 to 46, and even more preferably 0 to 16.

The connecting group in the branched polyglycerol-modified silicone according to the invention connecting a silicon atom in the silicone chain and the branched polyglycerol chain described above preferably contains oxyphenylene groups. A group represented by the following formula (8) [hereinafter, referred to as connecting group (8)] 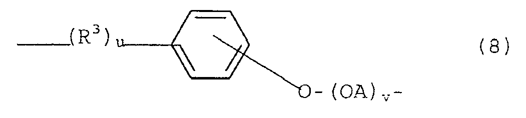 wherein,
- R³ is a straight- or branched-chain alkylene or alkenylene group having 1 to 22 carbons which may have one or more substituents;
- u is a number of 0 or 1;
- v is a number of 0 to 30;
- AO is the same as above;
- and v AO's may be the same or different from each other;
or a group represented by formula (9) [hereinafter, referred to as connecting group (9)] 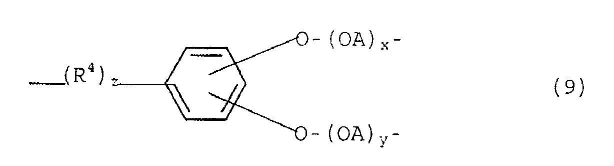 wherein,
- R⁴ is a straight- or branched-chain alkylene or alkenylene group having 1 to 22 carbons which may have one or more substituents;
- z is a number of 0 or 1;
- x is a number of 0 to 30;
- y is a number of 0 to 30;
- AO is the same as above;
- and x and y AO's may be the same or different from each other,
is preferable.

The connecting group (8) binds to a silicon atom in the silicone chain at the (R³)ᵤ side and to a branched polyglycerol chain at the (AO)ᵥ side. The connecting group (9) binds to a silicon atom in the silicone chain at the (R⁴)_{z} side and to branched polyglycerol chains at the (AO)ₓ and (AO)_{y} sides.

In the connecting groups (8) and (9), (R³)ᵤ and (R⁴)_{z} each are a group connecting a silicon atom in the silicone chain to the phenylene group of the oxyphenylene group contained in the connecting group of the invention; R³ and R⁴ each are preferably an alkylene or alkenylene group having 1 to 16 carbons, particularly preferably having 1 to 12 carbons; and examples thereof include ethylene, propylene, trimethylene, tetramethylene, pentamethylene, hexamethylene, heptamethylene, octamethylene, nonamethylene, decamethylene, undecamethylene, dodecamethylene, tridecamethylene, tetradecamethylene, pentadecamethylene, hexadecamethylene, and other groups. Among them, an ethylene, propylene or trimethylene group is more preferable, and an ethylene or trimethylene group is even more preferable.

Substituents which may be present on R³ and R⁴ include hydroxy, amino (having 1 to 22 carbons), imino (having 1 to 22 carbons), carboxy, alkoxy (having 1 to 22 carbons), and acyl (having 1 to 22 carbons) groups and the like. u and z may be 0 or 1, but are preferably 1.

In the connecting groups (8) and (9), AO is an oxyalkylene or oxyarylene group connecting a branched polyglycerol chain to the oxygen atom of the oxyphenylene group contained in the connecting group of the invention. AO is preferably an oxyethylene, oxypropylene or oxyphenylene group, and among them, an oxyethylene group is particularly preferable.

v, x and y each are a number of 0 to 30, preferably 0 to 15, more preferably 0 to 5, even more preferably zero from the viewpoint of easiness in synthesis and reaction. If v, x and y are not 0, v AO's, x AO's, and y AO's maybe the same or different from each other, and bound in any arrangement including alternating, block, and other regular sequences, or in a random manner.

In the connecting group (8), the position of two substituents, the oxygen atom and the (R³)ᵤ group (a silicon atom in the silicone chain, if u is 0), on the phenylene unit of the oxyphenylene group may be ortho, meta, or para, or a mixture thereof. In the connecting group (9), the position of either two of three substituents, two oxygen atoms and the (R⁴)_{z} group (a silicon atom in the silicone chain, if z is 0), on the phenylene unit of the oxyphenylene group may be ortho, meta, or para, or a mixture thereof, respectively.

Among the oxyphenylene group-containing connecting groups according to the invention, even more preferable is a connecting group represented by the following formula (10). [hereinafter, referred to as connecting group (10)]:

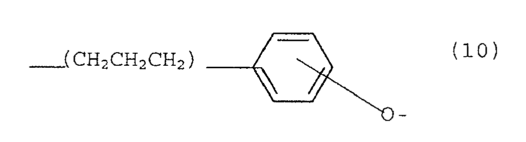

The connecting group (10) binds to a silicon atom in the silicone chain at the trimethylene side and to a branched polyglycerol chain at the oxygen atom side.

In the connecting group (10), the position of two substituents, the oxygen atom and the trimethylene group, on the phenylene unit in the oxyphenylene group may be ortho, meta, or para, or a mixture thereof, and ortho, para, or a mixture thereof is preferable.

The silicone constituting the branched polyglycerol-modified silicone according to the invention is a derivative of a polysiloxane having two or more silicon atoms, and the polysiloxane may have any structure such as straight-chain, branched-chain, or cyclic. The number-average molecular weight (*Mₙ*) of the polysiloxane is preferably 300 to 700,000, more preferably 300 to 200,000, and even more preferably 1,000 to 20,000. The number-average molecular weight can be determined by gel permeation chromatography (hereinafter, referred to as GPC), light scattering, or the like.

The branched polyglycerol-modified silicone according to the invention is preferably a straight-chain silicone represented by formula (11) [hereinafter, referred to as silicone (11)]: 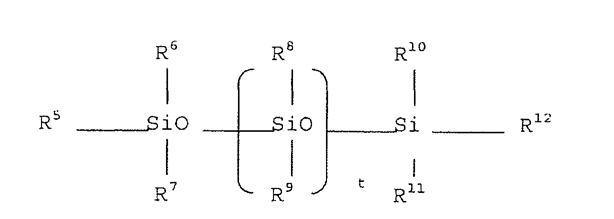 wherein,
- R⁵, R⁶, R⁷, t R⁸'s, t R⁹'s, R¹⁰, R¹¹, and R¹² each independently represent a connecting group to which a branched polyglycerol chain is connected, a straight- or branched-chain alkyl, alkenyl, or alkoxy group having 1 to 22 carbons which may have a substituent and may be substituted with fluorine atoms, or an aryl group having 6 to 22 carbons;
- at least one of R⁵, R⁶, R⁷, t R⁸'s, t R⁹'s, R¹⁰, R¹¹, and R¹² is a connecting group to which a branched polyglycerol chain is bound; and
- t is a number of 0 to 10,000.

In the silicone (11), R⁵, R⁶, R⁷, t R⁸'s, t R⁹'s, R¹⁰, R¹¹, and R¹² excluding the connecting group bound to a branched polyglycerol chain may be the same or different from each other and each are a straight- or branched-chain alkyl, alkenyl, or alkoxy group having 1 to 22 carbons, or an aryl group having 6 to 22 carbons which may have a substituent or be substituted with a fluorine atom. Examples of the alkyl groups having 1 to 22 carbons include methyl, ethyl, propyl, isopropyl, butyl, t-butyl, pentyl, hexyl, heptyl, octyl, nonyl, trifluoropropyl, and the like; examples of the alkenyl groups having 1 to 22 carbons, vinyl and allyl groups; and examples of the alkoxy groups having 1 to 22 carbons, methoxy, ethoxy, propoxy, butoxy, pentyloxy, hexyloxy, heptyloxy, octyloxy, phenoxy groups and the like. Among them, a straight- or branched-chain alkyl group having 1 to 12 carbons, vinyl group, allyl group, or aryl group having 6 to 12 carbons is preferable; an alkyl group having 1 to 3 carbons or phenyl group is more preferable; and a methyl, propyl, or phenyl group is even more preferable. Among them, a methyl group is even more preferable from the viewpoint of versatility, while a phenyl group is more preferable from the viewpoint of heat resistance.

In the silicone (11), the substituents which the groups R⁵ to R¹² may have, for example, phenyl, phenol, hydroxy, carboxy, amino (having 0 to 14 carbons), imino, (aminoethyl)amino, (dimethylaminoethyl)amino, polyoxyalkylene, mercapto, epoxy groups, and the like. A propyl group is particulary preferable as the base structure of R⁵ to R¹⁰ when they have above substituents.

In the silicone (11), at least one, preferably 1 to 10, even more preferably 1 to 5, and particularly preferably 1 to 2 of the groups R⁵, R⁶, R⁷, t R⁸'s, t R⁹'s, R¹⁰, R¹¹, and R¹² are the connecting groups bound to a branched polyglycerol chain. These connecting groups may be located at the side chain and/or one end or both ends of the silicone (11), or at the mixed sites thereof.

When one group selected from the group consisting of R⁵ to R⁷ and one group selected from the group consisting of R¹⁰ to R¹², are the connecting groups bound to a branched polyglycerol chain and the remaining groups of R⁵ to R⁷ and R¹⁰ to R¹², t R⁸'s, and t R⁹'s are the other groups, the branched polyglycerol-modified silicone according to the invention very preferably becomes a branched polyglycerol-modified silicone substituted at both ends, which easily forms a higher-order structure binding to each other in water or other solvents. In such a case, the other groups of R⁵ to R⁷ and R¹⁰ to R¹², t R⁸'s, and t R⁹'s each are preferably a methyl group.

When three or more connecting groups bound to a branched polyglycerol chain are present in t R⁸'s and t R⁹'s, the branched polyglycerol-modified silicone according to the invention preferably becomes a branched polyglycerol-modified silicone multi-substituted at the side chains, which is preferred according to the invention. Preferably, the branched polyglycerol-modified silicone according to the invention is a side-chain type polyglycerol-modified silicone.

t in the silicone (11) is a number of 0 to 10,000, preferably 1 to 3,000, even more preferably 5 to 500, and even more preferably 10 to 150.

The number-average molecular weight (*Mₙ*) of the branched polyglycerol-modified silicone according to the invention is preferably 500 to 500,000, more preferably 750 to 200,000, and even more preferably 1,000 to 100,000. The number-average molecular weight is determined by GPC (with the reference of standard polystyrene or polyethylene glycol).

The ratio of the total number (G) of groups (4), (5), (6), and (7) in the branched polyglycerol chains (hereinafter, referred to as the total number of glycerol groups) to the number of silicon atoms (Si) in the branched polyglycerol-modified silicone according to the invention, (G/Si), is preferably 0.001 to 50, more preferably 0.05 to 10, even more preferably 0.1 to 3, and even more preferably, 0.15 to 1.

In the branched polyglycerol-modified silicone according to the invention, the branched polyglycerol chain may have a small amount of ethyleneoxy and/or propyleneoxy groups. Said ethyleneoxy and/or propyleneoxy groups may be present randomly in the branched polyglycerol chain, or multiple ethyleneoxy and/or propyleneoxy groups may be present blockwise in consecutive chains in the branched polyglycerol chains. In that case, the blocks of multiple ethyleneoxy and/or propyleneoxy groups may be present either in the neighborhood of the connecting group of the branched polyglycerol chain or at the end or middle thereof. If the ethyleneoxy and/or propyleneoxy groups are present, the ethyleneoxy and/or propyleneoxy groups are present in an amount of preferably 0.001 to 0.5 molar equivalence and more preferably 0.02 to 0.2 molar equivalence with respect to 1 molar equivalence of the glycerol group.

The composition containing fragrance or perfume

It is preferred that the composition of the invention comprises, expressed as weight percentage,
(a) 0.01 to 10% of the fragrance or perfume
(b) 0.01 to 15% of the polysaccharide derivative
(c) 0.01 to 15% of the branched polyglycerol-modified silicone
(d) 1 to 60% one of more surfactants.

Preferably, the content of the fragrance or perfume, expressed as weight percentage, ranges from 0.1 to 5%, more preferably from 0.1 to 2% with respect to the composition.

Preferably, the content of the polysaccharide derivative, expressed as weight percentage, ranges from 0.01 to 5%, more preferably from 0.01 to 2%, with respect to the composition.

Preferably, the content of the branched polyglycerol-modified silicone, expressed as weight percentage, ranges from from 0.01 to 5%, more preferably from 0.1 to 2%, with respect to the composition.

Preferably, the content of the surfactant or mixture of surfactants, expressed as weight percentage, ranges from 5 to 35% with respect to the composition.

According to the invention, the surfactant (or active surface agent) can be an anionic, cationic, non-ionic or amphoteric surfactants, or mixtures thereof.

It is preferred that the weight ratio of the fragrance or perfume (component a) to the polysaccharide derivative (component b) is in the range from 60:1 to 1:10, preferably from 40:1 to 1:5, even more preferably from 30:1 to 1:2.

It is preferred that the weight ratio of the branched polyglycerol-modified silicone (component c) to the polysaccharide derivative (component b) is in the range from 60:1 to 1:10, preferably from 40:1 to 1:5, even more preferably from 30:1 to 1:2.

It is preferred that the weight ratio of the fragrance or perfume (component a) to the polyglycerol-modified silicone (component c) is in the range from 10:1 to 1:10, preferably from 5:1 to 1:5, even more preferably from 2:1 to 1:2.

The composition containing fragrance or perfume can be prepared in different ways, i.e. the fragrance or perfume (component a), the polysaccharide derivative (component b) and the branched polyglycerol-modified silicone (component c) can be added in situ in the surfactant base (component d).

Additionally, the fragrance or perfume (component a), the polysaccharide derivative (component b) and the branched polyglycerol-modified silicone (component c) can be mixed together separately from the surfactant base (component d) and added later.

Furthermore, the fragrance or perfume (component a) and the polysaccharide derivative (component b) can be mixed together to form a pre-mix, which is added later to the surfactant base (component d). The branched polyglycerol-modified silicone (component c) is added to the surfactant base (compoment d) before or after adding the pre-mix.

Additionally, the fragrance or perfume (component a) and the branched polyglycerol-modified silicone (component c) can be mixed together to form a pre-mix, which is added later to the surfactant base (component d). The polysaccharide derivative (component b) is added to the surfactant base (compoment d) before or after adding the pre-mix.

Finally, the fragrance or perfume (component a) and the polysaccharide derivative (component b) can be added to the surfactant base (component d) in the form of an oil-in-water emulsion. The branched polyglycerol-modified silicone (component c) can, either be added to the surfactant base (compoment d) before or after adding the oil-in-water-emulsion, or be also present in the oil-in-water emulsion.

### The oil-in-water emulsion

When preparing the composition of the present invention, the fragrance material or a perfume (component a) and the polysaccharide derivative (component b) are preferably added to the remaining ingredients in the form of an oil-in-water emulsion. This oil-in-water emulsion preferably comprises, expressed as weight percentage,
(a) 0.01 to 10% of the polysaccharide derivative,
(b) 1 to 50% of one or more water-soluble polyols different from the polysaccharide derivative
(c) 0.01 to 70% of one or more fragrances or perfumes
(d) 5 to 50% of water, and
(e) 0.01 to 30% of one or more emulsifiers,
with the proviso that the amounts add up to 100%.

Preferably, the content of the polysaccharide derivative in the oil-in-water emulsion, expressed as weight percentage, ranges from 0.1 to 5% with respect to the emulsion.

Preferably, the content of the water-soluble polyol in the oil-in-water emulsion, expressed as weight percentage, ranges from 5 to 45% with respect to the emulsion.

Preferably, the content of the fragrances or perfumes in the oil-in-water emulsion, expressed as weight percentage, ranges from 0.1 to 50% with respect to the emulsion.

Preferably, the content of the emulsifier in the oil-in-water emulsion, expressed as weight percentage, ranges from 0.1 to 20% with respect to the emulsion.

It is preferred that in the oil-in-water emulsion the weight ratio of the fragrance or perfume to the polysaccharide derivative is in the range from 60:1 to 1:10, preferably from 40:1 to 1:5, even more preferably from 30:1 to 1:2.

The oil-in-water emulsion may additionally include a hydrophobic material in which the fragrance or perfume can be dissolved or dispersed.

When using fragrances or perfumes which are solid at room temperature, the use of a hydrophobic material which is liquid at room temperature, as a solvent or dispersant, is advantageous.

The fragrance or perfume is chosen so that it may be emulsified in water at temperatures between its melting point and the boiling point of water.

The fragrance or perfume is preferably liquid at 20°C.

Said oil-in-water emulsion can be obtained by the process described in EP-A-1369102. Preferably, the oil-in-water emulsions are prepared by a process, which comprises the steps:
a) mixing together the polysaccharide, the water-soluble polyol, the fragrance or perfume (or part of the total amount of thereof), the emulsifier and part of the total amount of water, and finally (adding the remaining amount of fragrance or perfume, if necessary) and diluting the resulting mixture with the remaining amount of water, or
b) mixing together the polysaccharide and the fragrance or perfume (or part of the total amount of thereof), adding to said solution a mixture of water and the water-soluble polyol, adding to this solution the emulsifier, and finally (adding the remaining amount of fragrance or perfume, if necessary) diluting the resulting mixture with the remaining amount of water, or
c) mixing together the polysaccharide and the fragrance or perfume (or part of the total amount thereof), adding to said solution a mixture of water, the water-soluble polyol and the emulsifier, and finally (adding the remaining amount or fragrance of perfume, if necessary) diluting the resulting mixture with the remaining amount of water.

The composition of the present invention is preferably prepared by mixing the above-defined oil-in-water emulsion with the branched polyglycerol-modified silicone (component c). In a preferred embodiment, the resulting product is also present in the form of an oil-in-water-emulsion.

Said oil-in-water emulsion comprising the fragrance or perfume, the polysaccharide derivative and the branched polyglycerol-modified silicone can be obtained by the process described before. The branched polyglycerol-modified silicone can be added at any time to the pre-mixtures and mixtures described in the steps a), b) and c).

In this case, it is preferred that in the composition containing fragrance or perfume of the invention the oil-in-water emulsion comprises, expressed as weight percentage,
(a) 0.01 to 10% of the polysaccharide derivative,
(b) 1 to 50% of one or more water-soluble polyols different from the polysaccharide derivative
(c) 0.01 to 70% of one or more fragrances or perfumes
(d) 5 to 50% of water, and
(e) 0.01 to 30% of one or more emulsifiers,
(f) 0.01 to 70% of one or more branched polyglycerol-modified silicone
with the proviso that the amounts add up to 100%.

It is preferred that the weight ratio in the oil-in-water emulsion of the fragrance or perfume to the polysaccharide derivative is in the range from 60:1 to 1:10, preferably from 40:1 to 1:5, even more preferably from 30:1 to 1:2.

It is preferred that the weight ratio in the oil-in-water emulsion of the branched polyglycerol-modified silicone to the polysaccharide derivative is in the range from 60:1 to 1:10, preferably from 40:1 to 1:5, even more preferably from 30:1 to 1:2.

It is preferred that the weight ratio in the oil-in-water emulsion of the fragrance or perfume to the polyglycerol-modified silicone is in the range from 10:1 to 1:10, preferably from 5:1 to 1:5, even more preferably from 2:1 to 1:2.

The water-soluble polyol is different from the polysaccharide derivative and is preferably a polyhydric alcohol having two or more hydroxyl groups in its molecule. Specific examples can include ethylene glycol, propylene glycol, 1,3-butylene glycol, 1,4-butylene glycol, dipropylene glycol, polyethylene glycol with weight average molecular weights in the range from 100 to 1000, glucose, fructose, galactose, mannose, ribose, erythrose, maltose, maltitose, maltotriose, sucrose, xylitol, sorbitol, threitol, erythritol, glycerol, polyglycerol and starch alcohols. These water-soluble polyols can be used either alone or in combination. Particular preferred water-soluble polyols are ethylene glycol, propylene glycol, 1,3-butylene glycol, 1,4-butylene glycol, dipropylene glycol, polyethylene glycol with a weight average molecular weight in the range from 100 to 1,000, glycerol, polyglycerol, and mixtures thereof.

The oil-in-water emulsions can contain, additionally to the fragrance or perfume, to the polysaccharide derivative and to the branched polyglycerol-modified silicone, hydrophobic compounds like higher alcohols (having more than 5 carbons), sterols, silicones, fluorine-containing oils, (functional) oil components and the like, which are added to heighten the functions and additional values of toiletry products.

Suitable emulsifiers are, for example, nonionic surfactants from at least one of the following groups:
- products of the addition of 1 to 30 moles of ethylene oxide and/or 0 to 5 moles of propylene oxide onto linear C₈-C₂₂ fatty alcohols, C₁₂-C₂₂ fatty acids and alkyl phenols containing 8 to 15 carbons in the alkyl group and alkylamines containing 8 to 22 carbons in the alkyl group;
- alkyl and/or alkenyl oligoglycosides containing 8 to 22 carbons in the alkyl group and ethoxylated analogs thereof;
- addition products of 1 to 15 moles of ethylene oxide with castor oil and/or hydrogenated castor oil;
- addition products of 15 to 60 moles of ethylene oxide with castor oil and/or hydrogenated castor oil;
- partial esters of glycerol and/or sorbitan with unsaturated or saturated, linear or branched fatty acids containing 12 to 22 carbons and/or hydroxycarboxylic acids containing 3 to 18 carbon atoms and addition products thereof with 1 to 30 moles of ethylene oxide;
- mixtures of alkoxylated glycerides and alkoxylated glycerine as described in the European patent applications EP-A-0579887, EP-A-0586323, and EP-A-1045021, and are commercially available under the trademark LEVENOL^{®} and marketed by Kao Chemicals Europe
- partial esters of polyglycerol (average degree of selfcondensation 2 to 8), polyethylene glycol (weight average molecular weight 400 to 5000), trimethylolpropane, pentaerythritol, sugar alcohols (for example sorbitol), alkyl glucosides (for example methyl glucoside, butyl glucoside, lauryl glucoside) and polyglucosides (for example cellulose) with saturated and/or unsaturated, linear or branched fatty acids containing 12 to 22 carbons and/or hydroxycarboxylic acids containing 3 to 18 carbons and addition products thereof with 1 to 30 moles of ethylene oxide;
- mixed esters of pentaerythritol, fatty acids, citric acid and fatty alcohol and/or mixed esters of fatty acids containing 6 to 22 carbons, methyl glucose and polyols, preferably glycerol or polyglycerol;
- mono-, di- and trialkyl phosphates and mono-, di- and/or tri-PEG-alkyl phosphates and salts thereof;
- wool wax alcohols;
- polysiloxane/polyalkyl/polyether copolymers and corresponding derivatives,
- block copolymers, for example Polyethyleneglycol-30 Dipolyhydroxystearate;
- polymer emulsifiers;
- polyalkylene glycols;
- alkyl glyceryl ethers; and
- glycerol carbonate.

When the fragrance or perfume (component a) and the polysaccharide derivative (component b) are in the form of an O/W emulsion, the composition containing fragrance or perfume according to the invention comprises, expressed as weight percentage,
(i) 0.1 to 20%, preferably from 0.5 to 15% of the 0/W emulsion as described above comprising the fragrance material or a perfume and the polysaccharide derivative
(ii) 0.01 to 5%, more preferably from 0.1 to 2% of the branched polyglycerol-modified silicone
(iii) 0.01 to 50%, preferably from 0.5 to 25% of the surfactant or mixture or surfactants.

When the fragrance or perfume (component a), the polysaccharide derivative (component b) and the branched polyglycerol-modified silicone (component c) are in the form of an O/W emulsion, the composition containing fragrance or perfume according to the invention comprises, expressed as weight percentage,
(i) 0.1 to 35%, preferably from 0.5 to 15%, more preferably from 0.5 to 5%, of the O/W emulsion as described above comprising the fragrance material or a perfume, the polysaccharide derivative and the branched polyglycerol-modified silicone
(ii) 0.01 to 65%, preferably from 0.5 to 35% of the surfactant or mixture or surfactants.

### Personal Care compositions

The use of a composition containing fragrance or perfume according to the invention for enhancing the fragrance or perfume longevity of a personal care composition is also included in the subject of the present invention.

The use of a composition containing fragrance or perfume of the present invention in personal care compositions is particularly preferable in view of the improvement of the fragrance performance and the long lasting properties.

The present invention also provides personal care compositions comprising the composition containing fragrance or perfume according to the invention in which the composition containing fragrance or perfume is present in quantities of 1 to 50% by weight, preferably 1.5 to 35% by weight, even more preferably 2 to 25% by weight with respect to the total weight of the personal care compositions.

Surfactants useful as surfactant base in the personal care compositions herein include anionic surfactant, a non-ionic surfactant, a cationic surfactant, an amphoteric surfactant or zwitterionic surfactant or mixtures thereof.

Typical examples of anionic surfactants are soaps, preferably C₁₂-C₁₈ fatty acid soaps, like soaps derived from lauric acid, myristic acid, palmitic acid, stearic acid, isostearic acid, oleic acid or linoleic acid. Said soaps contain an appropriate cation, selected from the group consisting of an alkali metal, an alkaline earth metal, ammonium, an alkylammonium, an alkanolammonium or a glucammonium. Preferably the cation group is selected from the group consisting of sodium, potassium and triethanolamine. Preferred C₁₂-C₁₈ fatty acid soaps include triethanolamine stearate, triethanolamine palmitate, triethanolamine myristate, triethanolamine laurate, sodium stearate, sodium palmitate, sodium myristate, sodium laurate, potassium stearate, potassium palmitate, potassium myristate, and potassium laurate.

Other examples of examples of anionic surfactants are, alkylbenzenesulfonates, alkanesulfonates, olefin sulfonates, alkylether sulfonates, alkyl ether carboxylic acids and salts thereof, glycerol ether sulfonates, α-methyl ester sulfonates, sulfofatty acids, alkylsulfates, fatty alcohol ether sulfates, glycerol ether sulfates, hydroxy mixed ether sulfates, monoglyceride (ether) sulfates, fatty acid amide (ether) sulfates, mono- and dialkyl sulfosuccinates, mono- and dialkyl sulfosuccinamates, sulfotriglycerides, amide soaps, amide ether carboxylic acids and salts thereof, fatty acid isethionates, fatty acid sarcosinates, fatty acid taurides, N-acylamino acids such as, for example, acyl lactylates, acyl tartrates, acyl glutamates, acyl aspartates, alkyl oligoglucoside sulfates, protein fatty acid condensates (particularly wheat-based vegetable products) and alkyl (ether) phosphates.

One of the preferred anionic surfactant is sodium lauryl ether sulfate, particularly preferred is sodium lauryl ether sulfate having an average degree of ethoxylation of 1 to 3, preferably 1 to 2.5, most preferably 2 to 2.5.

Typical examples of cationic surfactants are quaternary ammonium salts (quats) and the quaternized derivatives of polyalkanolamine esters (esterquats). Examples of commercially available quats are: Quartamin^{®} AB (Behentrimonium Chloride), Quartamin^{®} 60W25 (Cetrimonium Chloride) and Quartamin^{®} ABK (Behentrimonium Chloride and Cetearyl Alcohol), all marketed by KAO Chemicals Europe.

Examples of commercially available esterquats are Quartamin^{®} BTC-131 (Behenoyl PG-Trimonium Chloride), marketed by KAO Chemicals Europe, and Tetranyl^{®} CO-40 (Dioleoylethyl Hydroxyethylmonium Methosulfate and Dipropylene Glycol) marketed by KAO Chemicals Europe.

Specific examples of non-ionic surfactants are mixtures of alkoxylated glycerides and alkoxylated glycerine, like the commercially available under the trademark LEVENOL^{®} marketed by KAO Chemicals Europe, alkoxylated trimethyolol propane, alkoxylated 1,2,3-trihydroxy hexane, alkoxylated pentaetrythritol, alkoxylated sorbitol, alkoxylated glycerol, alkoxylated glycerol fatty acid ester, alkoxylated trimethyolol propane fatty acid ester, alkoxylated 1,2,3-trihydroxy hexane fatty acid ester, alkoxylated pentaetrythritol fatty acid ester, alkoxylated sorbitol fatty acid ester, fatty alcohol polyglycol ethers, alkylphenol polyglycol ethers, fatty acid polyglycol esters, C₆-C₂₂ fatty alcohols, fatty acid amide polyglycol ethers, fatty amine polyglycol ethers, mixed ethers and mixed formals, optionally partly oxidized alk(en)yl oligoglycosides or glucuronic acid derivatives, fatty acid-N-alkylglucamides, protein hydrolyzates (particularly wheat-based vegetable products), polyol fatty acid esters, sugar esters, alkyl polyglucosides, sorbitan esters, polysorbates and alkanolamides, including alkoxylated alkanolamides and alkyl ether carboxylic acid alkanolamides.

Amphoteric surfactant includes ampholytes and betaines. Specific examples are alkyl amine oxides, alkyl betaines, alkyl sulphobetaines (sultaines), amidoalkyl betaines, alkyl glycinates, alkyl carboxyglycinates, alkyl amphoacetates, alkyl amphopropionates, alkylamphoglycinates, alkyl amidopropyl betaines, alkyl amidopropyl- and hydroxysultaines. Particularly preferred amphoteric surfactants are alkyl amine oxides, alkyl sulphobetaines (sultaines), alkylamphoglycinates alkyl amphoacetates such as sodium coco monoamphoacetate or sodium coco diamphoacetate, and alkyl amidopropyl betaines such as cocoamido propyl betaine.

The personal care compositions according to the present invention can also comprise, as further auxiliaries and additives, superfatting agents, pearlescent waxes, bodying agents, thickeners, polymers, silicone compounds, fats, waxes, stabilizers, biogenic active ingredients, deodorants, antiperspirants, antidandruff agents, film formers, UV light protection factors, antioxidants, preservatives, insect repellents, self-tanning agents, tyrosine inhibitors (depigmentation agents), solubilizers, perfume oils, dyes and the like.

The personal care compositions according to the present invention are particularly useful as body shampoos, hair shampoos, hair lotions, hair conditioners, shower preparations, bath preparations, liquid soaps and other cosmetic rinse-off products; as well as creams, body lotions, gels, and other cosmetic leave-on products. The products have a range of viscosities from 10 to 100,000 centipoise (cP) at 10 s⁻¹ shear rate, in the case of leave-on products and a a range of viscosities from 10,000 to 100,000 centipoise (cP) at 10 s⁻¹ shear rate in the case of rinse-off products.

It is preferred that the personal care composition comprising the composition containing fragrance or perfume is a hair conditioner. Hair conditioners are usually aqueous liquids containing a range of materials designed to improve the shine, body and manageability of the hair, the hair conditioning agents being present at 2-60%, typically 2-10% (as active matter) expressed as weight percertage based on the total composition. The conditioning materials consist of non-ionic, amphoteric or cationic polymers (for example hydroxyethyl cellulose, polyquarternium-39, guar hydroxypropyl-trimonium chloride, polyquarternium-10 and quaternised keratin), non-ionic, cationic and amphoteric surfactants (for example stearyl dimethyl benzyl ammonium chloride, ethoxylated fatty alcohols, ethoxylated esters of fatty alcohols and cetyl trimethyl ammonium bromide) and oils and waxes (for example cetearyl alcohol, silicone oils, mineral oils, natural oils such as avocado and jojoba oils, and glycerol esters). Suitable hair conditioners may also contain other ingredients, including solvents, vitamins, hair nourishing ingredients, dyes, preservatives and pH control agents. Hair conditioners can be designed either to be left on the hair after use or to be rinsed out.

The total amount of the fragrance or perfume is in the range of 0.1 to 5 % by weight, preferably from 0.3 to 2 % by weight, with respect to the total weight of the hair conditioner composition.

The total amount of the polysaccharide derivative is in the range of 0.01 to 3% by weight, preferably from 0.1 to 1.5% by weight with respect to the total weight of the hair conditioner composition.

The total amount of the branched polyglycerol-modified silicone is in the range of 0.01 to 5% by weight, preferably from 0.1 to 3% by weight, with respect to the total weight of the hair conditioner composition.

The total amount of the surfactant or mixture of surfactants is in the range of 1 to 25% by weight, preferably from 5 to 20% by weight with respect to the total weight of the hair conditioner composition.

### Household compositions

The use of a composition containing fragrance or perfume according to the invention for enhancing the fragrance or perfume longevity of a household composition is also included in the subject of the present invention.

The use of a composition containing fragrance or perfume of the present invention in household compositions is particularly preferable in view of the improvement of the fragrance performance and the long lasting properties.

The present invention also provides household compositions comprising the composition containing fragrance or perfume according to the invention in which the composition containing fragrance or perfume is present in quantities of 1 to 50% by weight, preferably 1.5 to 35% by weight, even more preferably 2 to 25% by weight with respect to the total weight of the household compositions.

The household compositions according to the present invention are particularly useful as laundry detergents, fabric softeners, soaps, all-purpose cleaners, bathroom cleaners, floor cleaners, hard surface cleaners and the like.

Surfactants useful as surfactant base in the househould compositions herein include well-known cationic, anionic, nonionic, amphoteric and zwitterionic surfactants or mixtures thereof. Typical of these are the alkyl benzene sulphonates, alkyl sulphonates, alkyl- and alkyl ether sulphates, primary alkyl sulphates, alkoxylated alcohols, alpha-sulphonates of fatty acids and of fatty acid esters, alkyl betaines, and alkyl polyglycosides all known in the detergent art.

It is preferred that the househould compositions comprising the surfactant base and the fragrance of perfume delivery system is a fabric softener. The main benefits delivered by such products are softness, fragrance and anti-static. Softness is usually the most important.

A fabric (textile) softener contains at least one softening agent, which functions to give the fabric a softer handle. Frequently such agents also provide an anti-static benefit. Such agents are usually cationic, but may be nonionic, amphoteric or zwitterionic materials.

Cationic surfactants that act as textile-softeners and that may constitute one of the components of the fabric softener compositions according the invention are well known by the skilled person.

Specific examples of fabric softening agents are acrylic quaternary ammonium compounds, diamido quaternary ammonium salts, ester quaternary ammonium salts (esterquats), quaternary imidazolinium salts and the like.

Among these, mention may be made of quaternary ammonium compounds whose hydrophobic chains are not interrupted by an ester group, for example those described in patents US-A-4719382 and US-A-4237016, of which the most well known is hydrogenated tallow dialkyldimethylammonium chloride, also known as DTDMAC, such as that marketed by KAO Chemicals Europe under the trademark QUARTAMIN ^{®} D86P.

However, according to the invention, the following esterquats, are preferred:
- the quaternised diesters of fatty acids with 1,2-dihydroxy-3-dimethylaminopropane, such as described by US-A-4137180 and European patent application EP-A-0585040,
- the quaternised diesters of fatty acids with N-methyldiethanolamine, such as those described in French patent application FR-A-1593921 and in European patent EP-B-0239910, for example the hydrogenated tallow diester quaternised with methyl chloride and marketed by Chemicals Europe under the trademark KAOSOFT^{®} PH,
- the amido ester salts of fatty acids with N-methyl-N-aminopropylethanolamine, for example that marketed by KAO Corporation Japan under the trademark KAOSOFT^{®} 1,
- the quaternised diesters of fatty acids with triethanolamine, such as those described in US-3915867 and in a large number of later patents, for example the diesters of partially hydrogenated tallow which are quaternised with dimethyl sulphate and which are marketed by KAO Chemicals Europe under the trademark TETRANYL^{®} AT-7590, TETRANYL^{®} L1/90 and TETRANYL^{®} L1/90S.

It should be pointed out that when reference is made to the term "diester" this is intended to indicate that the diester predominates in the mixture, although the product may always contain variable amounts of monoester compounds and, in the case of triethanolamine, triester compounds.

There also come into consideration as cationic surfactants having a softening character, the oligomeric cationic surfactants,
- esterquats derived from dicarboxylic acids, fatty acids and alkanolamines as described in the international patent application WO-A-9849132, for example those marketed by KAO Chemicals Europe under the references TETRANYL ^{®} PH-2 and TETRANYL^{®} PH-5,
- or esterquats obtained from alkanolamine esters based on the esterification reaction of optionally alkoxylated alkanolamines with dicarboxylic acids, fatty acids and fatty alcohols which are optionally alkoxylated, as described in the European patent application EP-A-1136471, for example those marketed by KAO Chemicals Europe under the references TETRANYL ^{®} CL-518.

The non-ionic surfactants that condition textiles or other fibres and that may be also present in the fabric softener according to the invention, are also well known to the person skilled in the art and of these there may be mentioned: fatty acids and their esters, especially those containing from 8 to 18 carbon atoms, which are linear or branched and alkoxylated or non-alkoxylated; alkoxylated or non-alkoxylated Guerbet alcohols; esters of glycerol and polyglycerol, for example HOSTACERIN DGMS and HOSTACERIN DGI marketed by Clariant; xylitol esters; alkoxylated or non-alkoxylated sorbitan esters, for example KAOPAN marketed by KAO Chemicals Europe; esters of sugars, such as glucose, fructose, galactose, mannose, xylose, arabinose, ribose, 2-deoxyribose and sucrose; C₆-C₂₂ fatty alcohols; mixtures of alkoxylated glycerides and alkoxylated glycerine, wich are commercially available under the trademark LEVENOL ^{®} marketed by KAO Chemicals Europe; ethoxylated polyglycerol esters, for example HOSTACERIN DGL and HOSTACERIN DGSB marketed by Clariant; alkyl polyglucosides, for example AG-10LK, marketed by Kao Corporation Japan; and alkoxylated or non-alkoxylated pentaerythritol esters, for example RADIA 7171 and RADIA 7176, marketed by Oleofina. Also exhibiting a good conditioning power are non-ionic surfactants with amide groups, among which there may be mentioned derivatives of amine, such as glucamine, for example MEDIALAN GAC and MEDIALAN GAL marketed by Clariant, and also derivatives of methylethanolamine, diethanolamine, isopropanolamine and monoethanolamine, with linear or branched fatty acids, especially C8-18 fatty acids. Finally, it can also be mentioned the esteramines described in the European patent application EP-A-443313.

Other non-ionic compounds which may be used as textile-conditioners are waxes, such as paraffins, microcrystalline waxes derived from petroleum, and synthetic waxes.

Of all the non-ionic surfactants described, the following are especially preferred: mixtures of alkoxylated glycerides and alkoxylated glycerine, sorbitan monoesters and pentaerythritol esters, especially those having a tallow, hydrogenated tallow, palm, behenic or oleic chain.

In referring to other optional components, without this having to be regarded as an exhaustive description of all possibilities, which, on the other hand, are well known to the person skilled in the art, the following may be mentioned:
a) other products that enhance the performance of the softening compositions, such as silicones, amine oxides, anionic surfactants, such as lauryl ether sulphate or lauryl sulphate, amphoteric surfactants, such as cocoamidopropyl betaine or alkyl betaines, sulphosuccinates, polyglucoside derivatives, etc.
b) stabilising products, such as salts of amines having a short chain, which are quaternised or non-quaternised, for example of triethanolamine, N-methyldiethanolamine, etc., and also non-ionic surfactants, such as ethoxylated fatty alcohols, ethoxylated fatty amines, ethoxylated alkyl phenols, etc.
c) products that improve viscosity control, for example inorganic salts, such as calcium chloride, magnesium chloride, calcium sulphate, sodium chloride, etc.; products which can be used to reduce viscosity in concentrated compositions, such as compounds of the glycol type, such as, for example, ethylene glycol, dipropylene glycol, polyglycols, etc.; and thickening agents for diluted compositions, for example, polymers derived from cellulose, guar gum, etc.
d) components for adjusting the pH, which is preferably from 1.5 to 4.5, such as any type of inorganic and/or organic acid, for example hydrochloric, sulphuric, phosphoric, citric acid etc.
e) agents that improve soil release, such as the known polymers or copolymers based on terephthalates.
f) bactericidal preservative agents, such as formol, Kathon GC, Bronopol, etc.
g) other products such as antioxidants, colouring agents, perfumes, germicides, fungicides, anti-corrosive agents, anti-crease agents, opacifiers, optical brighteners, pearl lustre agents, etc.

The fabric softeners can be obtained by simply mixing their components until they have been dispersed or dissolved, using methods well known by the person skilled in the art.

A fabric softening composition which is intended to be added during the washing cycle ar_d/or the rinsing cycle rinse may be in the form of a solid, a powder or tablet for instance.

According to the invention, the fabric softener is preferably in the form of a liquid, and is an aqueous dispersion in water. Such a fabric softener may contain from 1% to 15% weight of a fabric softening agent in the case of standard (diluted) fabric softener but may contain higher levels from up to 30% or even 40% by weight in the case of very concentrated fabric softeners. The composition will usually also contain water and other additives, which may provide the balance of the composition.

Liquid fabric softeners are customarily prepared by melting the softening ingredients and adding the melt to hot water, with agitation to disperse the water-insoluble ingredients.

The amount of perfume incorporated in the fabric softener according to the invention preferably lies in the range from 0.01% to 10% by weight with respect to the total weight of the fabric softener composition.

The total amount of the fragrance or perfume in fabric softeners according to the invention containing less than 15% by weight of fabric softening agent is preferably 0.05 to 1% by weight, more preferably from 0.1 to 0.8% by weight with respect to the total weight of the fabric softener composition.

The total amount of the fragrance or perfume in fabric softeners according to the invention containing more than 15% by weight of fabric softening agent (and up to 40% by weight) is preferably 0.05 to 3% by weight, more preferably from 0.1 to 2% by weight with respect to the total weight of the fabric softener composition.

The total amount of the branched polyglycerol-modified silicone in fabric softeners according to the invention is in the range of 0.01 to 5% by weight, preferably from 0.1 to 3% by weight, with respect to the total weight of the fabric softener composition.

The total amount of the surfactant or mixture of surfactants in fabric softeners according to the invention is in the range of 1 to 80% by weight, preferably from 5 to 55% by weight, with respect to the total weight of the fabric softener composition.

The following examples are given in order to provide a person skilled in the art with a sufficiently clear and complete explanation of the present invention, but should not be considered as limiting of the essential aspects of its subject, as set out in the preceding portions of this description.

### Examples

### Example 1. Fabric Softener

Fabric softener compositions shown in Table 1 were prepared according to following procedure.

Esterquat (softening agent), fatty alcohol, dispersant, the polysaccharide derivative and perfume were co-melt at 50°C. Water was heated to 50°C, and the co-melt was added to the water under stirring, at 150 rpm. Said mixture was stirred for 15 min.

Compositions A, B and C are comparative examples while composition 1 is according to the invention.

In the case of compositions containing the branched polyglycerol-modified silicon (comparative experiment C and composition 1), this component was pre-solved in ethanol (ratio 1:1) and added to the mixture 10 minutes after co-melt addition, stirring for a further 5 minutes to complete the 15 minutes dispersion time. Mixture was then cool down to 25 °C at a gradient of 1°C/min.

**Table 1. - Fabric Softener compositions (percentages referred to active matter)**

| | A | B | C | 1 (invention) |
|---|---|---|---|---|
| Esterquat¹ | 3.75 | 3.75 | 3.75 | 3.75 |
| Fatty Alcohol² | 1.0 | 1.0 | 1.0 | 1.0 |
| Dispersant³ | 0.25 | 0.25 | 0.25 | 0.25 |
| Branched polyglycerol-modified silicone⁴ | - - - | - - - | 0.15 | 0.15 |
| Polysaccharide derivative⁵ | - - - | 0.075 | 0 | 0.075 |
| Perfume⁶ | 0.3 | 0.3 | 0.3 | 0.3 |
| Deionized water | to 100 | to 100 | to 100 | to 100 |

| | | | | |
|---|---|---|---|---|
| ¹Tetranyl ® L1/90S (Di-alkyl ester of triethanol ammonium methyl sulphate) available from Kao Chemicals Europe ²Kalcol ® 6870 (cetearyl alcohol) available from Kao Chemicals Europe ³Levenol ® C-201 (INCI Name: Glycereth-17 cocoate) available from Kao Chemicals Europe ⁴Branched polyglycerol-modified silicone. Example 9 of EP-A-1489128 (p. 18-19) ⁵Polysaccharide derivative Example 11 of EP-A-1369102 (p. 11) ⁶Perfume FPEJ-37190-H-DPG available from Kao Chemicals Europe | | | | |

100% cotton towels were washed with a commercially available detergent (product name: Ariel, manufactured by Procter & Gamble) at a dosage of 2.68% by weight with respect the weight of the towels in a domestic washing machine at 60°C during approximately 50 minutes). Then, during a standard rinsing cycle, the compositions of Table 1 are applied on the towels. In both the washing and the rinsing cycle water of controlled hardness (20°f, Frech degrees) was used. The softener compositions were added at a dosage of 0.2% by weight with respect the weight of the of the towels.

Treated towels were smelled by an expert panel of 6 panellists to assess the perfume intensity, both on wet and on dry towels, by means of a triangular test comparing towels treated with comparative examples B, C or composition 1 against towels treated with comparative experiment A. Triangular test was carried out according to international standard ISO 6658:2005 (Section 6.2.3.), so for each pair of products under comparison, 6 different combinations to assess were presented.

On dry towels assessment was carried out after 24h, 5 days and 7 days after they had been treated with the softener compositions. During all test period towels were stored under controlled temperature and humidity conditions (20°C, 60%RH).

Results of sensorial perfume intensity assessment are given in Table 2.

**Table 2. - Scores to higher perfume intensity (number of positive answers from a total of 36)**

| | Wet | 24h | 5 days | 8 days |
|---|---|---|---|---|
| B vs A | 9 | 12 | 13 | 15 |
| C vs A | 7 | 11 | 16 | 18 |
| 1 vs A | 16 | 16 | 23 | 20 |

Composition 1 is showing a perfume intensity enhancement in wet conditions and also a positive effect on the odour longevity (sustantivity), demonstrating the synergetic effect of the combination of the polysaccharide derivative and the branched polyglycerol-modified silicone.

Additionally, perfume deposited onto towels was also analysed by SPME (Solid Phase Micro-Extraction) GC/MS (Gas Cromatography/Mass Spectrometry). Each sample was analyzed by triplicate.

For each analyzed sample total area of chromatogram was quantified. The % of area increase related to composition A was calculated. Results are summarized in Table 3.

**Table 3. - % of total area increase respect to composition A**

| | 24h | 5 days |
|---|---|---|
| B | 5 | 20 |
| C | 5 | 30 |
| 1 | 25 | 60 |

Analytical data confirm the synergistic effect of the combination of the polysaccharide derivative and the branched polyglycerol-modified silicone (composition 1) to enhance the perfume deposition during the application of fabric softeners on fabrics (textile fibres) and the odour longevity (sustantivity).

### Example 2. Hair conditioner

Hair conditioner compositions shown in Table 4 were prepared according to the following procedure.

Branched polyglycerol-modified silicone is added to the fatty alcohol (cetearyl alcohol). Said pre-mixture is heated up to melting point of the fatty alcohol. The cationic surfactant is added to water at 70°C and the pre-mixture between the fatty alcohol and the branched polyglycerol-modified silicone is added afterwards. The mixture is homogeneised at 250 rpm using a propeller mixer. After 30 minutes, the conditioner is allowed to cool down slowly (from 0.15 to 0.5 °C/min).

Perfume and the polysaccharide derivative in the form of an oil-in-water emulsion (composition 1) and perfume alone (comparative examples A and B) are added below 40°C and water is adjusted to 100%.

**Table 4 - Hair conditiner compositions (percentages referred to active matter)**

| | A | B | 1 (invention) |
|---|---|---|---|
| Cationic surfactant¹ | 1.5 | 1.5 | 1.5 |
| Fatty alcohol² | 3.0 | 3.0 | 3.0 |
| Branched polyglycerol-modified silicone³ | - - - | 1.0 | 1.0 |
| Perfume⁴ | 0.5 | 0.5 | - - - |
| Perfume and Polysaccahride derivative in the form of o/w emulsions ⁵ | - - - | - - - | 2.5 |
| Deionized Water | to 100 | to 100 | to 100 |

| | | | |
|---|---|---|---|
| ¹Quartamin^{®} 60W25 (cetrimonium chloride, 25% active matter) available from Kao Chemicals Europe ²Kalcol ® 6850 (cetearyl alcohol) available from Kao Chemicals Europe ³Branched polyglycerol-modified silicone. Example 9 of EP-A-1489128 ⁴Perfume SSSE-41104 available from Kao Chemicals Europe ⁵The o/w emulsion is prepared as described in Examples 1-11 (production of o/w emulsions) of EP-A-1369102 (p. 16) using the polysaccharide derivative Example 11 of EP-A-1369102 (p. 11). The content of the polysaccharide derivative in the o/w emulsion is 1% by weight and the content of the perfume is 20% by weight based on the total weight of the o/w emulsion. | | | |

Hair tresses of about 20 cm length and 2.5 cm width, with an approximate weight of 6 g are treated with 1 g of the hair conditioner compositions A, B and 1. Treated hair tresses were smelled by an expert panel of 6 panellists to assess the perfume intensity. Comparative examples A and B were compared as well as comparative experiment B and compositon 1 (according to the invention), using the Triangular test (according to international standard ISO 6658:2005, Section 6.2.3.). Every panelist was given 6 combinations for each pair of products.

Samples were evaluated straight after application (wet), after drying (4 hours) and after 24 hours. During all test period hair tresses were stored under controlled temperature and humidity conditions (20°C, 60% RH). Results of the Triangular tests are shown in Table 5:

**Table 5. - Scores to higher perfume intensity (number of positive answers from a total of 36)**

| | Wet | 4h | 24h |
|---|---|---|---|
| 1 vs A | 16 | 21 | 11 |
| 1 vs B | 15 | 19 | 14 |

Composition 1 shows a positive long lasting effect on perfume intensity when compared to the comparative examples.

Additionally, perfume deposited onto onto the hair surface afted treating hair tresses with 2 g of hair conditioners A, B and 1 was also analysed by SPME (Solid Phase Micro-Extraction) GC/MS (Gas Cromatography/Mass Spectrometry). Each sample was analyzed by triplicate.

For each analyzed sample total area of chromatogram was quantified. The % of area increase related to composition A was calculated. Results are summarized in Table 6.

**Table 6. - % of total area increase respect to composition A**

| | Wet | 24h |
|---|---|---|
| B | 17 | 8 |
| C | 33 | 28 |

Analytical data corroborate the synergistic effect of the combination of the polysaccharide derivative and the branched polyglycerol-modified silicone (composition 1) to enhance the perfume deposition during the application of hair conditioners on hair and the odour longevity (sustantivity), especially when the perfume and the polysaccharide derivative are in the form of an o/w emulsion.

## Claims

1. A composition containing fragrance or perfume comprising the following essential components
(a) a fragrance material or a perfume;
(b) a polysaccharide derivative derived from a polysaccharide, or a derivative thereof, by substituting a part or all of the hydrogen atoms of the hydroxyl groups of the polysaccharide or the derivative thereof with substituent group (A):
(A) groups represented by the following formula:
-E¹-(OX)ₙ-E²-R (1)
wherein
- E¹ represents a linear or branched, divalent saturated C₁-C₆ hydrocarbon group which may be substituted with one or more hydroxyl groups or oxo groups,
- X may be the same or different and each independently represent a divalent saturated C₁-C₆ hydrocarbon group, and n represents a number of from 8 to 300,
- E² represents an ether bond or an oxycarbonyl group (-OCO- or -COO-), and
- R represents a linear or branched C₄-C₃₀ alkyl group which may be substituted with one or more hydroxyl groups,
- the H(s) of one or more hydroxy groups in each of said substituted groups (A) may be further substituted with the groups of said substituted groups (A);
(c) a branched polyglycerol-modified silicone;
(d) a surfactant or mixture of surfactants.

2. A composition containing fragrance or perfume according to claim 1, wherein a part or all of the hydrogen atoms of the hydroxyl groups in the polysaccharide derivative are substituted with one or more groups selected from the following groups (B), (C) and (D):
(B) a C₁-C₅ sulfoalkyl group or salt thereof, each of which may be substituted with one or more hydroxyl groups,
(C) a C₂-C₆ carboxyalkyl group or salt thereof, each of which may be substituted with one or more hydroxyl groups, and
(D) a group represented by the following formula (2):
D¹-N(+)R¹R²R³ X(-) (2)
wherein
- D¹ represents a linear or branched, divalent, saturated C₁₋₆ hydrocarbon group which may be substituted with one or more hydroxyl groups
- R¹, R² and R³ may be the same or different and each independently represent a linear or branched C₁₋₃ alkyl group which may be substituted with one or more hydroxyl groups, and
- X(-) represents hydroxyl ion, a halogen ion or an organic acid ion,
in which each of the hydrogen atom(s) of one or more hydroxyl groups in these groups may be further substituted with the group (A), as defined in claim 1, (B), (C) or (D).

3. A composition containing fragrance or perfume according to claim 1 or 2, wherein the polysaccharide or a derivative thereof is selected from the group consisting of cellulose, starch, hydroxyethylcellulose, methylcellulose, ethylcellulose and hydroxypropylcellulose.

4. A composition containing fragrance or perfume according to claims 1 to 3, wherein the degree of substitution of the polysaccharide, or the derivative thereof, with the substituent group (A) is 0.0005 to 0.5 per constituent monosaccharide residual group.

5. A composition containing fragrance or perfume according to any of the preceding claims, wherein the recurring unit of the polysaccharide derivative is represented by the formula (3): 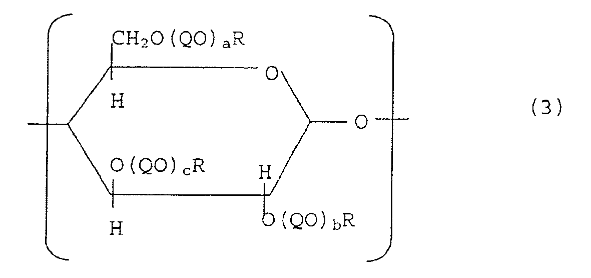 wherein
- R may be the same or different and each independently represents a group selected from
iii) hydrogen, methyl, ethyl, hydroxyethyl or hydroxypropyl
iv) a substituent group (A) of formula (1)
- E¹- (OX)ₙ - E² - R (1)
wherein E¹, X, E² and R have the same meaning as defined in claim 1;
and wherein,
- Q may be the same or different and each independently represents a C₂-C₄ alkylene group
- a, b, and c may be the same or different and each independently represent a number from 0 to 10
- the groups QO and R, as well as a, b,and c in the same recurring unit or in the different recurring units may be the same or different,
- the H(s) of one or more hydroxy groups in each of said substituted groups (A) may be further substituted with said substituted groups (A).

6. A composition containing fragrance or perfume according to any of the preceding claims, wherein the branched polyglycerol-modified silicone comprises at least one branched polyglycerol chain comprising at least one branched glycerol group represented by the below shown formula (4) and being connected via a connecting group to a silicon atom of the silicone 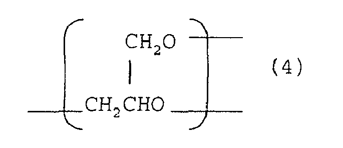 wherein, two oxygen atoms, independently of each other, bind to a glycerol or glycidol group represented by formula (4) above or the following formula (5), (6) or (7):

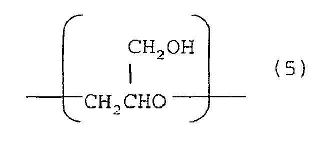

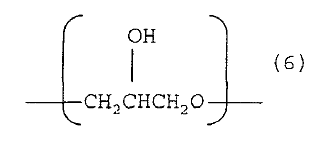

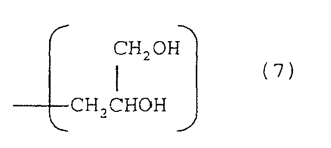

7. A composition containing fragrance or perfume according to claim 6, wherein in the branched polyglycerol-modified silicone the average total number of the groups selected from the glycerol and glycidol groups represented by formulae (4), (5), (6) or (7) in the branched polyglycerol chain is from 3 to 201.

8. A composition containing fragrance or perfume according to claim 6 or 7, wherein in the branched polyglycerol-modified silicone the connecting group is a group containing an oxyphenylene group.

9. A composition containing fragrance or perfume according to claim 8, wherein in the branched polyglycerol-modified silicone the connecting group is a group represented by formula (8) or (9) 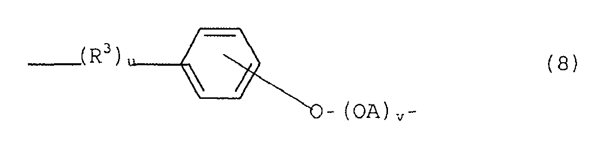 wherein,
- R³ is a straight- or branched-chain alkylene or alkenylene group having 1 to 22 carbons which may have one or more substituents;
- u is a number of 0 or 1;
- v is a number of 0 to 30;
- AO is an alkylenoxy group having 1 to 4 carbons (also called oxyalkylene group) or an arylenoxy group having 6 to 10 carbons (also called oxyarylene group);
- and v AO's may be the same or different from each other; and 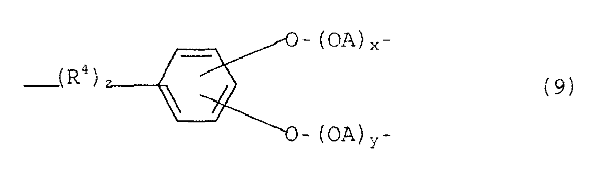
wherein,
- R⁴ is a straight- or branched-chain alkylene or alkenylene group having 1 to 22 carbons which may have one or more substituents;
- z is a number of 0 or 1;
- x is a number of 0 to 30;
- y is a number of 0 to 30;
- AO is the same as above;
- and x and y AO's may be the same or different from each other.

10. A composition containing fragrance or perfume according to claim 9, wherein in the branched polyglycerol-modified silicone the connecting group is a group represented by formula (10).

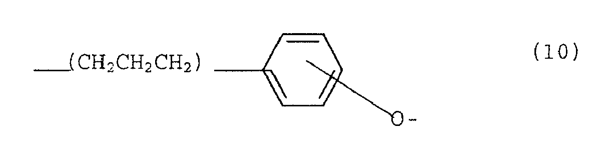

11. A composition containing fragrance or perfume according to any of the preceding claims, wherein the branched polyglycerol-modified silicone is a silicone represented by formula (11) 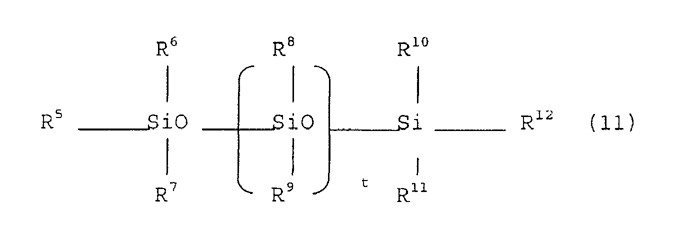 wherein,
- R⁵, R⁶, R⁷, t R⁸'s, t R⁹'s, R¹⁰, R¹¹, and R¹² each independently represent a connecting group to which a branched polyglycerol chain is connected, a straight- or branched-chain alkyl, alkenyl, or alkoxy group having 1 to 22 carbons which may have a substituent and may be substituted with fluorine atoms, or an aryl group having 6 to 22 carbons;
- at least one of R⁵, R⁶, R⁷, t R⁸'s, t R⁹'s, R¹⁰, R¹¹, and R¹² is a connecting group to which a branched polyglycerol chain is bound; and
- t is a number of 0 to 10,000.

12. A composition containing fragrance or perfume according to claim 11, wherein in the branched polyglycerol-modified silicone one group selected from the group comprising R⁵ to R⁷ and one group selected from the group comprising R¹⁰ to R¹² are connecting groups to which a branched polyglycerol chain is bound; and the other R⁵ to R⁷ and R¹⁰ to R¹², t R⁸'s, and t R⁹'s each are a methyl group.

13. A composition containing fragrance or perfume according to any of the preceding claims, wherein said composition comprises, expressed as weight percentage,
(a) 0.01 to 10% of the fragrance or perfume
(b) 0.01 to 15% of the polysaccharide derivative
(c) 0.01 to 15% of the branched polyglycerol-modified silicone
(d) 1 to 60% of one or more surfactants.

14. A composition containing fragrance or perfume according to any of the preceding claims, wherein the fragrance material or a perfume (component a) and the polysaccharide derivative (component b) are present in the form of an oil-in-water emulsion.

15. A composition containing fragrance or perfume according to claim 14, comprising an oil-in-water emulsion, a branched polyglycerol-modified silicone and one or more surfactants, wherein the oil-in-water emulsion comprises, expressed as weight percentage,
(a) 0.01 to 10% of the polysaccharide derivative,
(b) 1 to 50% of one or more water-soluble polyols
(c) 0.01 to 70% of one or more fragrance materials or perfumes
(d) 5 to 50% of water, and
(e) 0.01 to 30% of one or more emulsifiers,
with the proviso that the amounts add up to 100%.

16. A composition containing fragrance or perfume according to claim 14 or 15, wherein the weight ratio in the oil-in-water emulsion of the fragrance or perfume (component
a) to the polysaccharide derivative (component b) is in the range from 60:1 to 1:10.

17. A composition containing fragrance or perfume according to claims 1 to 13, wherein the fragrance or perfume (component a), the polysaccharide derivative (component b) and the branched polyglycerol-modified silicone (component c) are present in the form of an oil-in-water emulsion.

18. A composition containing fragrance or perfume according to claim 17, wherein the oil-in-water emulsion comprises, expressed as weight percentage,
(a) 0.01 to 10% of the polysaccharide derivative,
(b) 1 to 50% of one or more water-soluble polyols different from the polysaccharide derivative
(c) 0.01 to 70% of one or more fragrances or perfumes
(d) 5 to 50% of water,
(e) 0.01 to 30% of one or more emulsifiers,
(f) 0.01 to 70% of one or more branched polyglycerol-modified silicone
with the proviso that the amounts add up to 100%.

19. A composition containing fragrance or perfume according to claims 17 or 18, wherein in the oil-in-water emulsion the weight ratio of the fragrance or perfume (component a) to the polysaccharide derivative (component b) is in the range from 60:1 to 1:10.

20. A composition containing fragrance or perfume according to claims 17 to 19, wherein the weight ratio of the branched polyglycerol-modified silicone (component c) to the polysaccharide derivative (component b) is in the range from 60:1 to 1:10.

21. A composition containing fragrance or perfume according to claims 17 to 20, wherein the weight ratio of the fragrance or perfume (component a) to the polyglycerol-modified silicone (component c) is in the range from 10:1 to 1:10.

22. A composition containing fragrance or perfume according to claims 15 to 21, wherein in the oil-in-water emulsion the water-soluble polyol is selected from the group consisting of ethylene glycol, propylene glycol, 1,3-butylene glycol, 1,4-butylene glycol, dipropylene glycol, polyethylene glycol with a weight average molecular weight in the range from 100 to 1,000, glycerol, polyglycerol, and mixtures thereof.

23. A composition containing fragrance or perfume according to claims 15 to 22, wherein in the oil-in-water emulsion the emulsifier is selected from the group consisting of alkoxylated C_{S}-C₂₂ fatty alcohols, alkoxylated C₁₂-C₂₂ fatty acids, alkoxylated C₈-C₁₅ alkyl phenols and alkoxylated C₁₂-C₂₂ alkyl amines, containing from 2 to 15 moles of ethylene oxide; alkyl polyglucosides having 8 to 22, preferably 10 to 18, carbon atoms in the alkyl chain and containing from 1 to 20, preferably from 1.1 to 5, glucoside units; alkoxylated glycerides or mixtures of alkoxylated glycerides and alkoxylated glycerine; and alkyl glyceryl ethers.

24. A personal care composition comprising a composition containing fragrance or perfume as defined in claims 1 to 23.

25. A personal care composition according to claim 24, wherein said composition is a hair conditioner.

26. A household composition comprising a composition containing fragrance or perfume as defined in claims 1 to 23.

27. A household composition according to claim 26, wherein said composition is a fabric softener.

28. The use of a composition containing fragrance or perfume according to claims 1 to 23 for enhancing the fragrance or perfume longevity of a personal care composition.

29. The use according to claim 28, wherein said personal composition is a hair conditioner.

30. The use of a composition containing fragrance or perfume according to claims 1 to 23 for enhancing the fragrance or perfume longevity of a household composition.

31. The use according to claim 30, wherein said household composition is a fabric softener.
